# EUROPEAN PATENT SPECIFICATION

(11) **EP 0 371 736 B1**
(45) Date of publication and mention of the grant of the patent: **13.04.1994**
(21) Application number: 89312302.6
(22) Date of filing: 28.11.1989
(51) Int. Cl.: A61L 15/26, A61L 15/28, A61L 15/32, A61L 15/44

(54) **Wound covering**
Wundverband
Pansement pour blessures

(30) Priority: 29.11.1988 JP 301856/88
(43) Date of publication of application: 06.06.1990
(73) Proprietor: MITSUBISHI KASEI CORPORATION, Tokyo 100 (JP)
(72) Inventor: Shioya, Nobuyuki, Yokohama-shi kanagawa-ken (JP); Kuroyanagi, Yoshimitsu, Hachioji-shi Tokyo (JP); Kounami, Yasuo, Sagamihara-shi Kanagawa-ken (JP); Kobayashi, Tatsuhiko, Sagamihara-shi Kanagawa-ken (JP)
(74) Representative: Woods, Geoffrey Corlett

(56) References cited:
- EP-A- 0 184 233
- DE-A- 2 631 909
- US-A- 3 800 792

## Description

This invention relates to wound coverings.

In particular, it relates to the wound coverings which comprise two layers, one being a polyurethane resin film which has good moisture permeability and the other being a sheet of a biopolymeric material, which is effective for growth of tissue cells as an artificial skin.

Some properties necessary for wound coverings are: 1) adhesion, 2) flexibility, 3) durability, 4) easy handling, 5) preservation, 6) sterile filtration, 7) compatibility with cells, 8) blood stanching, and 9) water-vapour permeability.

However, although conventional wound coverings satisfy some of these properties, they do not satisfy all the properties.

For example, a sheet consisting of only a biopolymeric material substantially lacks flexibility and sterile filtration. Due to the bad flexibility, the sheet lacks adhesion at a wound surface, and some sheets substantially lack preservation property, and cannot be handled easily. In the case of poor sterile filtration, it is necessary to apply a cream containing an antibacterial agent every 2 or 3 days to the diseased part. This is very troublesome for the nurse and very painful for the patient.

On the other hand, a laminated sheet of a biopolymeric material sheet and a silicone film has a poor water-vapour permeability, and exudate is dangerously stored under the covering. It is possible to make such a laminated sheet thinner to try to overcome this defect, but a thinner sheet lacks easy handling.

Generally a lot of exudate is generated at the first stage of using a wound covering. A laminate sheet can have holes made mechanically in it to drain the exudate, but when the generation of exudate becomes small the holes become clogged by solidification of the exudate. Therefore, after clogging of the holes, it is necessary for the sheet to have good water-vapour permeability. For example, in the case of a burn injury, water-loss by evaporation from a wound is 300 g H₂O/m²·24 hrs for a first degree burn, 4300 g H₂O/m²·24 hrs for a second degree burn and 3400 g H₂O/m²·24 hrs for a third degree burn (L.O. Lamke Burns. 3 p 159 - 165).

A silicone-resin film used as a wound covering, for example, does not have sufficient water-vapour permeability (it has a low water-vapour permeability of 1000 - 1700 H₂O/m²·24hrs). Therefore, no wound covering has been provided until now which has a water-vapour permeability sufficient for the large amount of evaporation mentioned above in addition to good flexibility, durability and easy handleability.

EP-A-184,233 discloses a drug dispensing wound dressing comprising a polyurethane matrix that is the reaction product of (A) an isocyanate terminated prepolymer formed by reaction of isophorone diisocyanate and a macroglycol, and (B) a monomer containing hydroxyl and vinyl groups.

DE-A-2,631,909 discloses a multilayer membrane for use as a synthetic skin which comprises a first layer formed from a material which does not provoke an immune response and which is insoluble and nondegradable in the presence of body fluids and/or body enzymes and a second layer formed from a nontoxic material which controls the moisture flux of the overall membrane to about 0.1 to 1mg/cm²/hr.

US-A-3,800,792 discloses a surgical dressing comprising a thicker layer of a collagen compressed foam film to which has been laminated, without any adhesive, a thin continuous layer of an inert polymer material, such as polyurethane, having a moisture vapour transmission rate of slightly higher than that of human skin.

The present inventors have made studies to solve the problems of conventional sheets which consist of only biopolymeric materials and a laminate sheet thereof, i.e. to provide a wound covering which is excellent in 1) adhesion, 2) flexibility, 3) durability, 4) easy handling, 5) preservation, 6) sterile filtration, 7) compatibility with cells, 8) blood stanching, and 9) water-vapour permeability.

The present invention provides a wound covering comprising a sheet of a biopolymeric material and a film of a polyurethane resin, which film has a water-vapour permeability of 2,000 g/m²·24hrs or more, and which polyurethane resin is obtainable by reacting a diisocyanate and a random copolymer of tetrahydrofuran and ethylene oxide to produce a urethane prepolymer, and extending the molecular chain of the said prepolymer by using a chain extender, the said random copolymer containing 20 to 80% by weight of the ethylene oxide unit in the molecular chain thereof and having a number-average molecular weight of 800 to 3000.

The polyurethane resin used for the polyurethane film in the present invention is obtainable by using a random copolymer of tetrahydrofuran (hereinafter referred to as "THF") and ethylene oxide (hereinafter referred to as "EO") as the polyol component. This copolymer may be synthesized by ring-opening copolymerization of THF and EO at temperature of 20 to 50°C, in the presence of an initiator, for example water, short-chain diols such as ethylene glycol and 1,4-butane diol, and a Lewis acid-catalyst such as boron trifluoride ethyl etherate.

The content of the EO unit in the random copolymer of THF and EO is 20 to 80 weight %, preferably 30 to 70 weight %, more preferably 40 to 60 weight % based on the weight of the copolymer in order to reduce swelling by absorbing water and lowering in physical properties of the polyurethane film. The number-average molecular weight of the random copolymer of THF and EO is 800 to 3000. If the number-average molecular weight is less than 800, the polyurethane film becomes hard, and if more than 3000, it has large tacky adhesion and an increase of swelling by absorbing water. In order to provide excellent film properties, the number-average molecular weight is preferably 1000 to 2500.

A mixture of the random copolymer of THF and EO and polytetramethylene ether glycol (hereinafter referred to as "PTMG") can also be used if necessary. In this case, the number-average molecular weight of the PTMG to be added is 800 to 3000, and that of the polyol mixture is 800 to 3000, preferably 1000 to 2500, in order to provide well-balanced properties.

There is no disadvantage in the present invention if the above random copolymer of THF and EO includes a partial block-copolymer-structure in its molecular chain.

A copolymer of THF and EO generally includes a block copolymer which is synthesized by the addition reaction of EO and PTMG obtained from the ring-opening polymerization of THF, or the addition reaction of THF and polyethylene glycol (hereinafter referred to as "PEG") obtained from ring-opening polymerization of EO. However, polyurethane produced from these block copolymers is not suitable for practical use, because the increase of swelling by absorbing water is remarkable with an increase of EO content, similar to the case of using a mixture of PTMG and PEG, due to the inclusion of the strong hydrophilic homopolymer long-chains of EO in the molecular chain.

When the above random copolymer of THF and EO, or the mixture of this random copolymer and PTMG, is used as the polyol component of a polyurethane, the polyurethane film has good water-vapour permeability as compared with the above block copolymer of THF and EO, or a mixture of PTMG and PEG used together, though the increase of the water absorption with increase of the EO unit content is small.

In order to obtain the polyurethane resin used in the present invention, in general there is used a method wherein, after obtaining a urethane prepolymer having an isocyanato group at the end of its molecular chain by reacting the above specific polyol component and an excess equivalent of diisocyanate at 70 to 120°C, the chain of the urethane prepolymer is extended by a chain extender at 20 to 100°C in an organic solvent.

The equivalent ratio of diisocyanate and polyol component in the urethane prepolymer is usually 1.5 to 6:1 preferably 1.8 to 4.5:1, to provide good physical properties and water-vapour permeability.

The isocyanates used are, for example, aromatic diisocyanates such as 4,4'-diphenylmethane diisocyanate, 2,4- and 2,6-tolylene diisocyanate, 1,5-naphthalene diisocyanate and m- and p-phenylenediisocyanate, alicyclic diisocyanates such as isophorone diisocyanate, 4,4'-dicyclohexylmethane diisocyanate, 1,4-cyclohexylene diisocyanate and hydrogenated tolylene diisocyanate, and aliphatic diisocyanates such as hexamethylene diisocyanate.

Among these, the alicyclic diisocyanates are preferred due to their resistance to yellowing and good mechanical properties. These diisocyanates are generally used alone, but two or more diisocyanates can be used together. Among the alicyclic diisocyanates 4,4'-dicyclohexylmethane diisocyanate is the most preferred due to its good balance of mechanical properties and water-vapour permeability of the film formed.

The chain extenders used are, for example, low molecular diols such as ethylene glycol, propylene glycol, diethylene glycol, 1,4-butanediol and 1,6-hexanediol, aliphatic diamines such as ethylenediamine, 1,2-propanediamine, tetramethylenediamine and hexamethylenediamine, alicyclic diamines such as isophoronediamine, 4,4'-dicyclohexylmethanediamine, 3,3'-dimethyl-4,4'-dicyclohexylmethanediamine and 1,4-cyclohexylenediamine, hydrazine hydrate and water.

Among these compounds, alicyclic diamines are the most preferred due to the good stability of the polyurethane solution and good heat resistance of the film formed. These compounds can be used alone, or two or more can be used together. The above low molecular diols may be used together in an amount such that the mechanical properties and heat-resistance of the film produced are not lowered too much. Among the alicyclic diamines isophoronediamine is the most preferred due to the solution stability and good balance of various properties of the film.

Organic solvents having a strong dissolving power, for example dimethylformamide, dimethylacetamide and dimethyl sulfoxide, are suitable for the synthesis of the polyurethane. These solvents can be used alone, and also used together with one or more solvents selected from aromatic compounds such as toluene and xylene, ketones such as methyl ethyl ketone, acetone and cyclohexanone, esters such as ethyl acetate and butyl acetate, ethers such as tetrahydrofuran and dioxane, and alcohols such as methanol and 2-propanol.

The polyurethane resin obtained as described above preferably contains the ethylene oxide unit in an amount of 15 to 60 weight % based on the total weight of the polyurethane. When the ethylene oxide unit content is lower than 15 weight %, the water-vapour permeability of the film is not sufficient, and when the ethylene oxide unit content is higher than 60 weight %, considerable dimensional change and lowering of physical properties take place by swelling when absorbing water.

If necessary a catalyst generally used for promoting the urethanization reaction, for example tertiary amines such as triethylenediamine and organic tin compounds such as dibutyl tin dilaurate, can be used in the reaction.

There may be previously contained in the polyurethane one or more of hindered phenol type antioxidant, benzophenone type or benzotriazole type ultra-violet absorbing agent or hindered amine type stabilizer in order to provide better durability. In this case, each additive may be added in an amount of 0.05 to 3 weight % based on the weight of the solid parts of the polyurethane, preferably in an amount of 0.2 to 1 weight % to obtain a better effect. In the polyurethane the above hindered amine type stabilizers are especially effective for avoiding oxidation deterioration upon sterilization treatment and property degradation by hydrolysis.

Either porous or non-porous polyurethane films are also available. It is well known that polyurethane films with a high water-vapour permeability are obtained when making the film porous.

Examples of preparation methods for obtaining porous polyurethane films are:
(1) a wet film-formation method wherein a solution of polyurethane resins is coated on a support, and soluble substances such as solvents are extracted in a coagulating bath; and
(2) a method wherein a water in oil-type emulsion is coated on a support, and the porous membrane is obtained after drying by heating.

The non-porous polyurethane films can be obtained by a dry film-formation method wherein a solution of a polyurethane resin is coated on a support or release paper and dried by heating. By this method, films can be obtained which have stable and reproducible water-vapour permeability, and also have sufficient practical strength, elongation, and durability even if used as a single film.

When dry film-formation is carried out, the supports used are not limited, but polyethylene or polypropylene films, release papers or clothes coated with fluoro- or silicone-type releasing agent are preferably used. The above release papers preferably have a uniform thickness, because the degree of the water-vapour permeability of the polyurethane films is in inverse correlation to the film thickness. The coating method is not specially limited; either a knife coater or a roll coater can be used. The drying temperature is arbitrarily set up according to the capacity of the drying instrument, but it is necessary to select a range of temperature so that insufficient drying or rapid solvent-removal does not occur. The temperature is preferably 60 to 130°C.

The thickness of the polyurethane films is usually 10 to 200 µm, preferably 10 to 80 µm. If the thickness is less than 10 µm, pin holes are likely to be formed when coating and the films formed are not easily handled because of blocking of the films. If the thickness is over 80 µm, it is difficult to obtain sufficient water-vapour permeability. The thickness dependence of the water-vapour permeability is small in the polyurethane films used in the present invention in comparison with other urethane-type films.

The polyurethane films used in the present invention have a high water-vapour permeability which is 2000 g/m²·24 hrs or more (by the measurement of JIS Z 0208), preferably 3000 g/m²·24 hrs or more for non-porous films having a thickness of 10 to 80 µm. A film having a water-vapor permeability of less than 2000 gm²·24 hrs causes discomfort by steam generated when the skin is covered with the film. The 100% modulus of the polyurethane film used in the present invention is 20 kg/cm² or more, preferably 30 kg/cm² or more. If the 100% modulus is less than 20 kg/cm², the films are likely to block each other. On the other hand, if it is more than 80 kg/cm², they are likely to lack flexibility and have reduced water-vapor permeability.

Since there is much exudation of body fluid in the first stage of using a wound covering, the recovery of a wound is slow because of the inhibition of adhesion of the wound covering with the wound by retention of the exudate between the wound and the wound covering, if the wound covering has insufficient water-vapour permeability.

To avoid slow recovery, it is preferred to provide holes in the wound covering, especially in the polyurethane film, in order to drain the exudate from a wound in the first stage.

It is sufficient for the holes to drain therethrough the exudate from an early stage wound. For this purpose, the diameter of the holes is about 0.01 to 1 mm, preferably about 0.1 to 0.7 mm, and the number of the holes is about 2 to 50/cm², preferably about 5 to 20/cm².

The holes are formed by sticking needles into the film or punching.

The holes may be blocked after the drain therethrough of the exudate in the early stage. It is rather preferred that the holes are blocked after the drain of the exudate in order to prevent penetration of bacteria. Therefore, it is also preferred to form slits which are easily opened and closed in place of holes. The slits may be formed in shapes such as '+', 'V', 'L', 'U', 'I' and '-'. The exudate can be drained through the slits, and thereafter the slits may be tightly closed to prevent penetration of bacteria.

When providing holes which are always open by needles, it is preferred to pre-heat the needles to the melting point or more of the polyurethane film. In this case, it is preferred to add an antibacterial agent into the polyurethane film and the sheet of biopolymeric materials in order to prevent penetration of bacteria. When using antibacterial agents, the diameter of the holes is preferably within the diameter of the inhibition zone of the antibacterial agent used.

As the biopolymeric material usable in the present invention, collagen, gelatin, alginic acid, chitin, chitosan, fibrin, dextran and polyamino acid may, for example, be mentioned. Of the above materials, collagen and chitosan are most preferably used. The collagen may include, for example, succinyl collagen, acetyl collagen and methyl collagen.

It is especially preferred that atelocolagen, which is obtained from removing non-helix parts at the end of the molecule by treating with a protease except for collagenase, is used, since it is near neutral and biocompatible.

Chitosan is also preferrably used. As chitosan, chitin deacetylated by the action of concentrated alkali is preferably used. The degree of deacetylation is 40% or more, preferably 50% or more.

It is also preferred that N-acylchitosan obtained by the reaction of chitosan with a carboxylic anhydride is used. A mixture of collagen and chitosan can also be used.

A sheet-formation method of the biopolymeric material is exemplified below:
A solution, dispersion or gel of collagens and/or chitosans is prepared. Then it is made into a sponge-like sheet by freeze-drying, or a film by removing solvents after flow-casting on a flat plate.

The thickness of the sheet of biopolymeric material is usually 10 to 100 µm, preferably 10 to 80 µm. When laminate sheets are formed with a polyurethane film, either a single sheet or two or more sheets of biopolymeric material can be piled together for use. It is also possible to obtain a wound covering having an antibacterial effect by incorporating antibacterial agents. To incorporate antibacterial agents into the wound covering of the present invention a sheet of biopolymeric material or a polyurethane film containing a given amount of an antibacterial agent may be used. A polyurethane film containing a given amount of an antibacterial agent may also be provided between the polyurethane film and the sheet of biopolymeric material, thereby forming a three-layered wound covering of a polyurethane film/polyurethane film containing antibacterial agent/sheet of biopolymeric material. Examples of the antibacterial agent are sulfa drugs, cephalosporins, penicillins and nalidixic acid and derivatives thereof. The amount of the antibacterial agent used is dependent on the type of the antibacterial agent used; However, it is usually 0.001 to 10% by weight based on the weight of the polyurethane film or the weight of the sheet of biopolymeric material.

The polyurethane film containing the antibacterial agent can be obtained by dissolving a polyurethane resin and an antibacterial agent in a solvent which is capable of dissolving both the resin and the agent, mixing the solution well, and then making it into a film.

As the solvent for dissolving both the resin and the agent, alcohols are usually used. Of the alcohols, those having 5 or less carbon atoms and a boiling point of not higher than 120°C are preferred. Methanol, ethanol, n-propanol, 2-propanol, n-butanol, sec-butanol, isobutanol and 3-pentanol may in particular be mentioned.

Examples of methods for laminating are a method wherein the sheet of biopolymeric material is laminated on the polyurethane film after swelling the surface of the polyurethane film with an organic solvent which can swell the film surface moderately, for example, alcohols such as methanol, and dried, and a method wherein the polyurethane film is laminated on the sheet of biopolymeric material after absorbing the solvent described above in the sheet thereby swelling the surface of the polyurethane film, and dried.

The wound covering thus obtained is used by contacting the surface of the sheet of biopolymeric material to a wound. With the cure of a wound, the biopolymeric material is taken in living tissue, and therefore the polyurethane film can be easily peeled off from the sheet without hurting the surface of a wound after cure.

The present invention further explained in the following Examples.

### Preparation Examples 1 - 4

### The preparation of THF-EO random copolymer

Random copolymerization of THF and EO was carried out in an autoclave for the compositions shown in Table 1, under the conditions of ordinary pressure, and a temperature of 30°C. Ethylene glycol was used as the initiation agent, and boron trifluoride ethyl etherate was used as the acid catalyst. After the completion of copolymerization, the acid catalyst in the product was neutralized with alkali, the precipitates were filtrated, and then dried by blowing dry nitrogen gas at 100°C.

Four random copolymers of THF and EO (hereinafter referred to as "polyol"), A, B, C, and D, were obtained. These are all colourless and transparent liquids, the number-average molecular weight and EO content being shown in Table 1.

The number-average molecular weight is calculated from the OH value, and the EO content is calculated from the amounts of EO charged.

**Table 1**

| No. of preparation examples | | 1 | 2 | 3 | 4 (comparative example) |
|---|---|---|---|---|---|
| Composition of polyols | | A | B | C | D |
| Components of polyol (Weight parts) | Ethylene glycol | 28.2 | 17.2 | 13.4 | 15.5 |
| | THF | 236 | 241.4 | 194.6 | 72.7 |
| | E O | 236 | 241.4 | 291.9 | 411.8 |
| | boron trifluoride ethyl etherate | 32.3 | 19.7 | 15.3 | 17.8 |
| Number-average molecular weight EO content (weight %) | | 1100 | 1800 | 2300 | 2000 |
| | | 50 | 50 | 60 | 85 |

### Preparation Examples 5 - 8

### Preparation of polyurethane film

### a) Preparation of polyurethane solution

A urethane prepolymer having an isocyanate group at the end of the molecular chain thereof was obtained by the reaction of 4,4'-dicyclohexylmethane diisocyanate and each of the polyols A, B, C, and D obtained in Preparation Examples 1 to 4 in the compositions a shown in Table 2 in a flask under an atmosphere of dry nitrogen at 100°C for 6 hours. After this, a chain-extending reaction for each of the above prepolymers was carried out in dimethylformamide using isophorone diamine as the chain extender for 2 hours at 30°C. Polyurethane solutions, which were each colourless, transparent and viscous, were obtained. The solids content of each solution was 25 weight %. The viscosities of the solutions at 25°C were 35,000 cps, 20,000 cps, 15,000 cps and 16,000 cps respectively.

### b) Formation of polyurethane film

Polyurethane resin was precipitated by dropping each of the above solutions into water. After filtrating and drying the solid polyurethane, methanol solutions which contained 15% by weight of the polyurethane resin were prepared. Silver sulfadiazine, as an antibacterial agent, was then added in an amount of 30 weight % based on the weight of the polyurethane resin, and the mixture was stirred to make it uniform.

Each polyurethane solution containing the antibacterial agent was flow-casted on glass plates provided with a spacer, expanded by a glass stick to uniform thickness, and dried for a whole day and night at 80°C. Colourless and transparent polyurethane films were obtained by a dry method. The film thickness was controlled to be about 30 µm by the spacer. In order to investigate the dependency of water-vapor permeability on film thickness, films with different thickness as in the range of 10 to 100 µm were prepared. The films obtained were preserved in a thermohygrostat at 23°C and 60% RH.

Property tests of the polyurethane films were carried out as follows.

Property test of the polyurethane film.

### 〈Measurement of increase of dimension by water-absorbing〉

The increase of dimension by water-absorbing was calculated from the measured values before and after water-absorbing, by soaking polyurethane films of 50 mm x 50 mm in water at 23°C for 24 hours.

### 〈Measurement of water-vapor permeability〉

The water-vapour permeability was obtained from measurement of the weight using a cup for water-vapour permeability measurement under the conditions of 40°C and 90% RH according to JIS Z-0208.

### 〈Oxidation resistance test〉

The influence to mechanical properties by oxidative deterioration was examined by irradiating γ rays at an exposure dose of 2.5 Mrad at a dose rate of 10⁶ rad/hour in air, supposing sterilization treatment by radiation.

### 〈Hydrolysis resistance test〉

Hydrolysis resistance was evaluated by measuring the mechanical properties after exposing the polyurethane films to an atmosphere of 70°C and 95% RH for 3 weeks.

### Preparation Example 9

### Preparation of polyurethane film

A hindered amine type stabilizing agent, Tinuvin® 622 LD (sold from Chiba-Geigy Co.), was added to the polyurethane solution obtained in Preparation Example 6 in an amount of 0.5 weight % based on the weight of polyurethane contained in the solution. Then, by the same method as in Preparation Example 6, a polyurethane film was prepared, and tests were carried out.

As shown in Table 4, the properties of the polyurethane film were remarkably improved in comparison with the film of Preparation Example 6 which does not contain Tinuvin® 622 LD, for example, in oxidative deterioration when irradiating γ rays and lowering of mechanical properties by hydrolysis.

**Table 2**

| No. of preparation example | | 5 | 6 | 7 | 8 (Comparative example) | 9 |
|---|---|---|---|---|---|---|
| Composition of polyurethane (Weight parts) | Polyol A | 305.7 | | | | |
| | Polyol B | | 359.6 | | | 359.6 |
| | Polyol C | | | 383.0 | | |
| | Polyol D | | | | 370.0 | |
| | 4,4'-dicyclohexylmethane diisocyanate | 145.6 | 104.7 | 87.3 | 96.9 | 104.7 |
| | Isophorone diamine | 48.7 | 35.7 | 29.7 | 33.1 | 35.7 |
| Additives | Tinuvin® 622LD | - | - | - | - | 2.5 0.5 wt% based on polyurethane |

### Preparation Examples 10 - 13

### Preparation of polyurethane film

Polyurethane solutions were prepared in the compositions shown in Table 3. In Preparation Examples 10 to 12, wherein 4,4-dicyclohexylmethane diisocyanate and isophorone diamine were used, the polyurethane solutions were prepared in the same manner as in Preparation Examples 5 to 8. The solid contents and viscosities at 25°C of the solutions were 25 weight % and 20,000 cps in Preparation Example 10, 20 weight % and 23,000 cps in Preparation Example 11 and 20 weight % and 18,000 cps in Preparation Example 12.

In Preparation Example 13, a urethane prepolymer was obtained by the reaction of 4,4'-diphenylmethane diisocyanate and polyol B in a flask under an atmosphere of dry nitrogen at 80°C for 4 hours. Then, a chain-extending reaction of the above prepolymer was carried out in dimethylformamide at 80°C for 6 hours using 1,4-butanediol as the chain extender in the presence of 100 ppm of dibutyl tin dilaurate. The solids content was 25 weight % and the viscosity at 25°C was 19000 cps.

The formation of the polyurethane film and tests of properties were carried out in the same manner as in Preparation Examples 5 to 8.

The properties of the polyurethane films of Preparation Examples 10 and 11 are shown in Table 4.

**Table 3**

| No. of preparation examples | | 10 | 11 | 12 (comparative example) | 13 |
|---|---|---|---|---|---|
| Composition of polyurethanes (weight parts) | Polyol | 330.5 | 305.8 | | 310.3 |
| | PTMG 2000 (note) | | | 369.8 | |
| | 4,4'-dicyclohexylmethane diisocyanate | 120.3 | 133.5 | 96.9 | |
| | 4,4'-diphenylmethane diisocyanate | | | | 150.9 |
| | Isophorone diamine | 49.2 | 60.7 | 33.3 | |
| | 1,4-butanediol | | | | 38.8 |
| Note) Polytetramethylene ether glycol having a number-average molecular weight 2000 (sold by Mitsubishi Kasei Corporation) | | | | | |

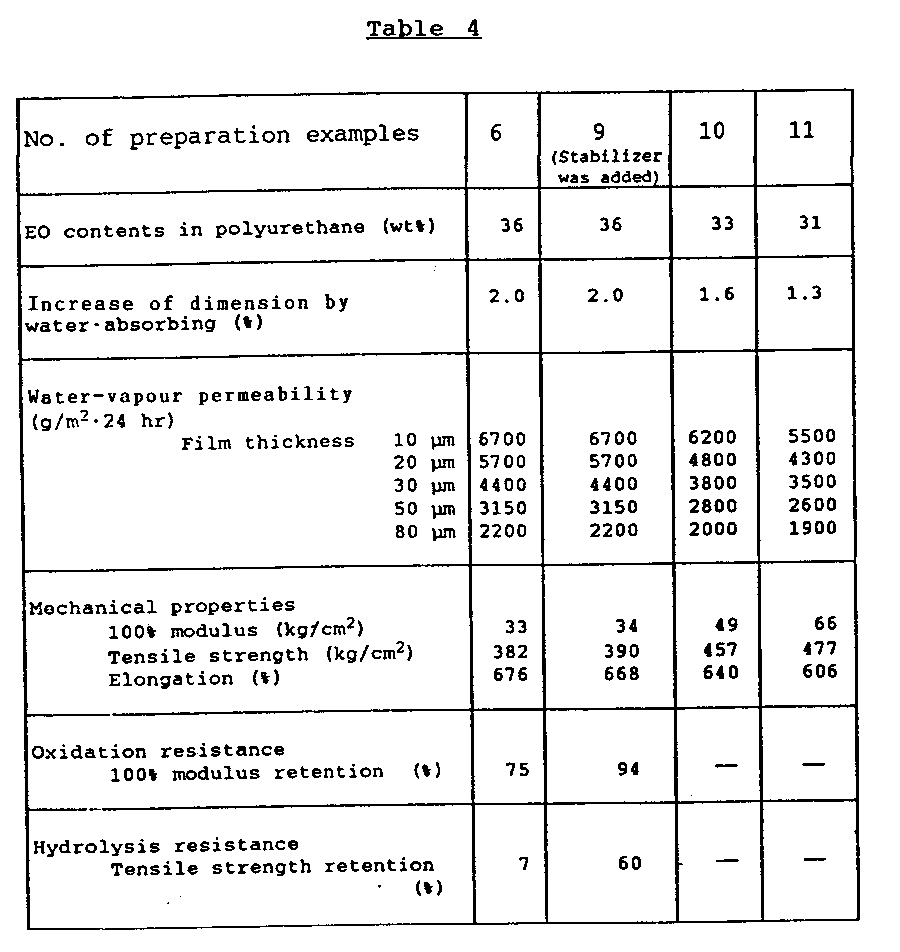

### Examples 1 - 6

A laminate sheet of a collagen sheet and a polyurethane film was prepared as follows. A uniform foaming solution was prepared by stirring a solution containing 1% by weight of atelocollagen, which was adjusted to pH 4 with hydrochloric acid in a homogenizer at a rate of 1500 rpm for 5 minutes.

It was flow-casted to a thickness of 1 to 2 mm. Then, the atelocollagen solution was neutralized by allowing it to stand in an atmosphere of ammonia for 30 minutes to be gelatinized. A sponge-like collagen sheet was subsequently obtained by freeze-drying the atelocollagen gel in a freeze-drier at -30°C. This collagen sheet was made water-insoluble by cross-linking by UV irradiation. A small amount of methanol was absorbed into the collagen sheet. Then, a laminate sheet of the sponge-like collagen sheet and the polyurethane film was obtained by laminating the collagen sheet with a polyurethane film having a thickness of 30 µm, obtained in Preparation Examples 5 - 7, 10, 11 or 13, and drying. A porous laminate sheet was obtained by forming holes with a frog.

The sheet was sterilized with gaseous ethylene oxide, and was tested:
A wound (diameter: 35 mm) of full thickness skin defect was made surgically at the abdominal part of a rat (SP rat, 6 to 8 weeks old), and a stainless ring of 0.4 mm⌀ was sutured (16 stitches). The laminate sheet obtained above was put on the ring, a moisture-retentive pad and sanitary cotton were placed on it, and then bandaged with an elastic band. The biocompatibility and regeneration of the dermis were histologically examined after one week and two weeks. The effect of the laminate sheet was also evaluated. These results are shown in Table 5. The curing effect is good in all the laminate sheets, and it has been found that the laminate sheet of the present invention is suitable for a wound covering.

### Example 7

A laminate sheet of an alginic acid sheet and a polyurethane film was prepared as follows. A uniform forming dispersion was obtained by dispersing sodium alginate (sold by Kimitsu Kagaku Industry Co., Ltd.) in water in a concentration of 2% and stirring the dispersion using a homogenizer at a rate of 1500 rpm for 5 minutes.

It was flow-casted to a thickness of 1 to 2 mm, and a sponge-like sheet of sodium alginate was obtained by freeze-drying at -80°C. Then, after spraying methanol on the sodium alginate sheet, the polyurethane film having a thickness of 30 µm obtained in Preparation Example 10 was laminated thereon, and dried to obtain a laminate sheet. A porous laminate sheet was obtained by forming holes with a frog. This was tested after sterilization treatment by gaseous ethylene oxide. The applicability of this laminate sheet as a wound covering was examined by an experiment similar to that used in Examples 1 to 6. As seen from the results in Table 5, the laminate sheet was found to be effective as a wound covering in the same manner as in the case of Examples 1 to 6.

### Example 8

A laminate sheet of a collagen-chitosan sheet and a polyurethane film was prepared as follows.

Into 0.5 liters of a 2% aqueous acetic acid solution were dissolved 9 g of N-succinylchitosan having a degree of succinylation of 50% and 1 g of atelocollagen 1 liter of methanol was then added into the solution.

12g of acetic anhydride was added to the aqueous methanol-acetic acid solution containing N-succinylchitosan and atelocollagen, mixed well and allowed to stand for 10 hours at room temperature. The solution was washed thoroughly with water to remove acetic acid and methanol, thereby obtaining 180 g of hydrated gel of acetylated chitosan-collagen.

The thus prepared hydrated gel was immersed in 3 liters of methanol, quick-freezed by adding dry ice and then freeze-dried in vacuo to obtain 10 g of a sponge of acetylated chitosan-collagen. The sponge was sliced into a piece of 2mm thickness, followed by irradiation of 5 Mrad γ-ray to cross-link the acetylated chitosan-collagen. The cross-linked sponge was immersed in methanol, then after removing the majority of methanol, a polyurethane film having a thickness of 25 µm obtained in Preparation Example 7 was laminated thereon and air dried at room temperature.

The thus obtained laminate sheet of the acetylated chitosan-collagen was provided with holes having a diameter of 0.3 mm (10 holes per 1 cm² area) by using a flog.

The porous laminate sheet was sterilized with gaseous ethylene oxide, and tested by the same tests as in Examples 1 to 7.

The results are shown in Table 5.

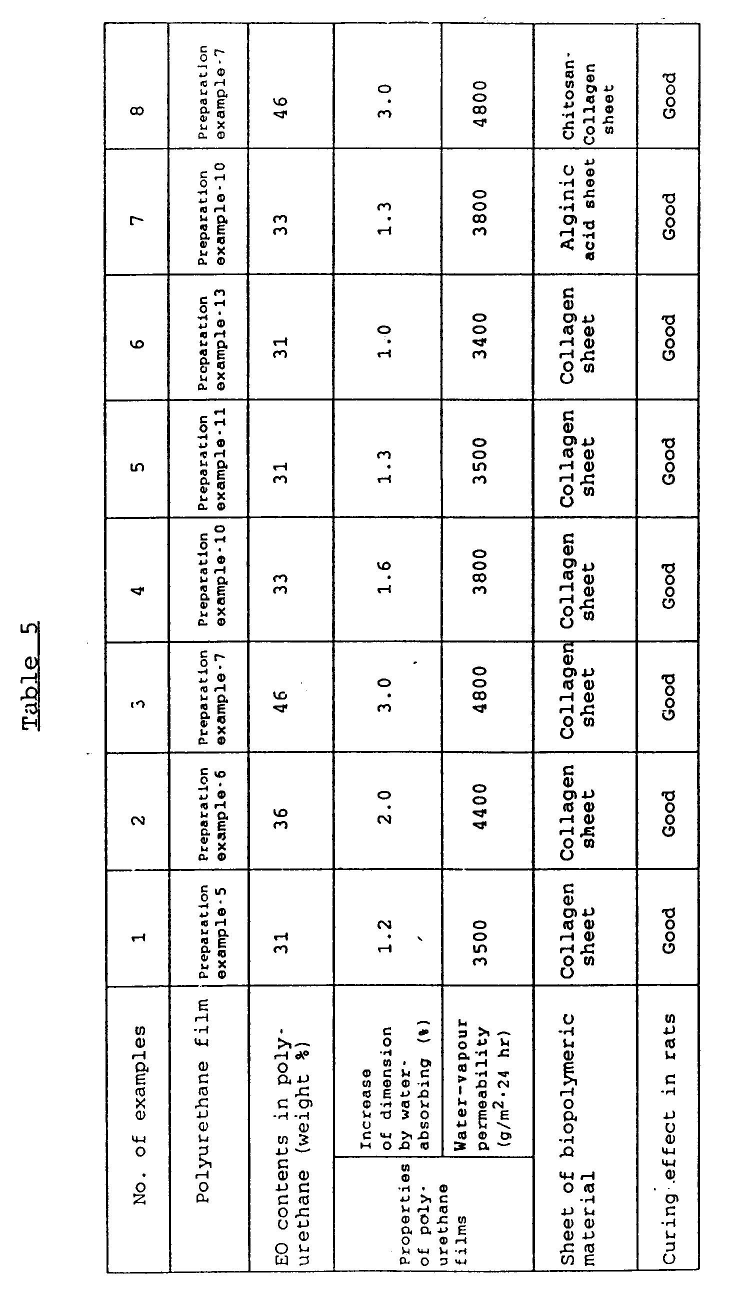

### Comparative Examples 1 - 2

Two porous laminate sheets were obtained from the sponge-like collagen sheet and the polyurethane films having a thickness of 30 µm obtained in Preparation Examples 8 and 12, in the same manner as in Examples 1 to 6. The applicability of these laminate sheets as wound coverings was examined. The results are shown in Table 6.

In Comparative Example 1, the water-vapor permeability of the polyurethane film was high, but adhesion between the laminate sheet and the wound was bad by swelling of the film due to water-absorbing. In Comparative Example 2, a curing effect was recognized, but the degree of the effect was insufficient compared with Examples 1 - 6. This was because water-vapor permeability of the polyurethane film was low.

**Table 6**

| No. of comparative examples | | 1 | 2 |
|---|---|---|---|
| Polyurethane film | | Preparation example - 8 | Preparation example - 12 |
| EO contents in polyurethane (weight %) | | 63 | 0 |
| Properties of urethane films | Increase of dimension by water-absorbing (%) | 15.0 | 0.7 |
| | Water-vapour permeability (g/m²·24 hr) | 6000 | 2300 |
| Sheet of biopolymeric material | | Collagen sheet | Collagen sheet |
| curing effect | | Bad adhesion by swelling, improper. | Effect showed, but inferior to examples. |

### Example 9

A laminate sheet of a polyurethane film and a collagen sheet containing an antibacterial agent was prepared as follow.

A foaming-dispersion was prepared by adding silver sulfadiazine into a 1% atelocollagen solution, which was adjusted to pH 4 with hydrochloric acid, in such an amount that the concentration of silver sulfadiazine was 1500 ppm, then stirring the solution using a homogenizer at a rate of 1500 rpm for 5 min.

It was flow-casted to a thickness of 1 to 2 mm. Then, the atelocollagen solution was neutralized by allowing it to stand in an atmosphere of ammonia for 30 min to be gelatinized. The atelocollagen gel was freeze-dried in a freeze-drier at -30°C to obtain a sponge-like collagen sheet. The collagen sheet was made water-insoluble by cross-linking by UV irradiation.

A small amount of methanol was absorbed in the collagen sheet. Then, the polyurethane film having a thickness of 30 µm obtained in Preparation Example 5 was laminated on the collagen sheet to obtain a laminate sheet of the polyurethane film and the collagen sheet containing an antibacterial agent.

A porous laminate sheet was obtained by forming holes using a frog.

After sterilization with gaseous ethylene oxide, the sheet thus obtained was tested as follows:
Pseudomonas aeruginosa was inoculated in an agar culture medium at a density of 10⁷ P.a/cm². Then, the sterilized laminate sheet was placed thereon, and the number of bacteria under the sheet was measured after one day.

As a comparison, the same experiment was repeated using a chitin-nonwoven fabric and a pig skin.

After one day, Pseudomonas aeruginosa under the sheet containing an antibacterial agent has been disinfected completely, whereas, under the chitin-nonwoven fabric and the pig skin, Pseudomonas aeruoinosa proliferated and the number of the bacteria was 10⁹ P.a/cm².

## Claims

1. A wound covering comprising a sheet of a biopolymeric material and a film of a polyurethane resin which film has a water-vapour permeability of 2,000 g/m²·24hrs or more, and which polyurethane resin is obtainable by reacting a diisocyanate and a random copolymer of tetrahydrofuran and ethylene oxide to produce a urethane prepolymer, and extending the molecular chain of the said prepolymer by using a chain extender, the said random copolymer having 20 to 80% by weight of the ethylene oxide unit in the molecular chain thereof and having a number-average molecular weight of 800 to 3000.

2. A wound covering according to Claim 1, wherein the film of a polyurethane resin or the sheet of a biopolymeric material contains an antibacterial agent.

3. A wound covering according to Claim 1 or 2, wherein the random copolymer has 30 to 70% by weight of the ethylene oxide unit in the molecular chain thereof.

4. A wound covering according to claim 1, 2 or 3 wherein the number-average molecular weight of the random copolymer is from 1000 to 2500.

5. A wound covering according to any one of the preceding claims wherein the diisocyanate is an alicyclic diisocyanate.

6. A wound covering according to Claim 5, wherein the alicyclic diisocyanate is selected from isophorone diisocyanate, 4,4'-dicyclohexylmethane diisocyanate, 1,4-cyclohexylene diisocyanate, and hydrogenated tolylene diisocyanates.

7. A wound covering according to any one of the preceding claims wherein the chain extender is an alicyclic diamine.

8. A wound covering according to Claim 7, wherein the alicyclic diamine is selected from isophorone diamine, 4,4'-dicyclohexylmethane diamine, 3,3'-dimethyl-4,4'-dicyclohexylmethane diamine, and 1,4-cyclohexylene diamine.

9. A wound covering according to any one of the preceding claims wherein the thickness of the film of a polyurethane resin is from 10 to 80 µm.

10. A wound covering according to any one of the preceding claims wherein the 100% modulus of the film of a polyurethane resin is 20 kg/cm² or more.

## Patentansprüche

1. Wundverband, umfassend eine Lage aus einem biopolymeren Material und einen Polyurethanharzfilm, der eine Wasserdampfpermeabilität von 2.000 g/m² x 24 Stunden oder mehr hat, wobei das Polyurethanharz erhältlich ist durch Umsetzung eines Diisocyanats und eines statistischen Copolymers aus Tetrahydrofuran und Ethylenoxid zur Herstellung eines Urethanprepolymers, und Verlängerung der Molekülkette dieses Prepolymers unter Verwendung eines Kettenverlängerers, wobei das statistische Copolymer 20 bis 80 Gew.-% Ethylenoxideinheiten in seiner Molekülkette enthält und ein Molekulargewichtszahlenmittel von 800 bis 3000 hat.

2. Wundverband gemäß Anspruch 1, dadurch **gekennzeichnet**, daß der Polyurethanharzfilm oder die Lage aus biopolymerem Material ein antibakterielles Mittel enthält.

3. Wundverband gemäß Anspruch 1 oder 2, dadurch **gekennzeichnet**, daß das statistische Copolymer in seiner Molekülkette 30 bis 70 Gew.-% Ethylenoxideinheiten hat.

4. Wundverband gemäß Anspruch 1, 2 oder 3, dadurch **gekennzeichnet**, daß das Molekulargewichtszahlenmittel des statistischen Copolymers 1000 bis 2500 beträgt.

5. Wundverband gemäß einem der vorhergehenden Ansprüche, dadurch **gekennzeichnet**, daß das Diisocyanat ein alicyclisches Diisocyanat ist.

6. Wundverband gemäß Anspruch 5, dadurch **gekennzeichnet**, daß das alicyclische Diisocyanat ausgewählt ist aus Isophorondiisocyanat, 4,4'-Dicyclohexylmethandiisocyanat, 1,4-Cyclohexylendiisocyanat und hydriertem Tolylendiisocyanat.

7. Wundverband gemäß einem der vorhergehenden Ansprüche, dadurch **gekennzeichnet**, daß das Kettenverlängerungsmittel ein alicyclisches Diamin ist.

8. Wundverband gemaß Anspruch 7, dadurch **gekennzeichnet**, daß das alicyclische Diamin ausgewählt ist aus Isophorondiamin, 4,4'-Dicyclohexylmethandiamin, 3,3'-Dimethyl-4,4'-dicyclohexylmethandiamin und 1,4-Cyclohexylendiamin.

9. Wundverband gemäß einem der vorhergehenden Ansprüche, dadurch **gekennzeichnet**, daß die Dicke des Polyurethanharzfilms 10 bis 80 µm beträgt.

10. Wundverband gemäß einem der vorhergehenden Ansprüche, dadurch **gekennzeichnet,** daß der 100%-Modul des Polyurethanharzfilms 20 kg/cm² oder mehr beträgt.

## Revendications

1. Pansement pour blessure comprenant une feuille d'une matière biopolymère et un film de résine de polyuréthanne, ce film ayant une perméabilité à la vapeur d'eau de 2 000 g/m².24 h ou plus, et cette résine de polyuréthanne pouvant être obtenue en faisant réagir un diisocyanate et un copolymère statistique de tétrahydrofuranne et d'oxyde d'éthylène afin de produire un prépolymère d'uréthanne, et en étendant la chaîne moléculaire de ce prépolymère à l'aide d'un agent d'extension de chaîne, le copolymère statistique comportant de 20 à 80 % en poids de motifs dérives d'oxyde d'éthylène dans sa chaîne moléculaire, et ayant un poids moléculaire moyen en nombre de 800 à 3 000.

2. Pansement pour blessure selon la revendication 1, dans lequel le film d'une résine de polyuréthanne ou la feuille d'une matière biopolymère, contient un agent antibactérien.

3. Pansement pour blessure selon la revendication 1 ou 2, dans lequel le copolymère statistique comprend de 30 à 70 % en poids de motifs dérivés d'oxyde d'éthylène dans sa chaîne moléculaire.

4. Pansement pour blessure selon la revendication 1, 2 ou 3, dans lequel le poids moléculaire moyen en nombre du copolymère statistique est de 1 000 à 2 500.

5. Pansement pour blessure selon l'une quelconque des revendications précédentes, dans lequel le diisocyanate est un diisocyanate alicyclique.

6. Pansement pour blessure selon la revendication 5, dans lequel le diisocyanate alicyclique est choisi parmi le diisocyanate d'isophorone, le diisocyanato-4,4'-dicyclohexylmethane, le diisocyanate de 1,4-cyclohexylène et les diisocyanates de tolylène hydrogénés.

7. Pansement pour blessure selon l'une quelconque des revendications précédentes, dans lequel l'agent d'extension de chaîne est une diamine alicyclique.

8. Pansement pour blessure selon la revendication 7, dans lequel la diamine alicyclique est choisie parmi l'isophorone diamine, la 4,4'-dicyclohexylméthane diamine, la 3,3'-diméthyl-4,4'-dicyclohexylméthane diamine et la 1,4-cyclohexylène diamine.

9. Pansement pour blessure selon l'une quelconque des revendications précédentes, dans lequel l'épaisseur du film de résine de polyuréthanne est de 10 à 80 µm.

10. Pansement pour blessure selon l'une quelconque des revendications précedentes, dans lequel le module à 100 % du film de résine de polyuréthanne est de 20 kg/cm² ou plus.
